# EUROPÄISCHE PATENTANMELDUNG

(11) **EP 3 069 821 A2**
(43) Veröffentlichungstag der Anmeldung: **21.09.2016**
(21) Anmeldenummer: 16151277.7
(22) Anmeldetag: 14.01.2016
(51) Int. Cl.: B24B 31/06, B22F 3/105, B22F 3/24, B22F 7/08, G02B 23/24, C23C 24/04

(54) **VERFAHREN ZUR FERTIGUNG EINES BAUTEILS**

(30) Priorität: 17.03.2015 DE 102015204801
(71) Anmelder: MTU Aero Engines AG, 80995 München (DE)
(72) Erfinder: Göpfert, Dagobert, 81476 München (DE)

(57) **Zusammenfassung**

Die Erfindung betrifft ein Verfahren zur Fertigung eines Bauteils, insbesondere eines Bauteils einer Strömungsmaschine, umfassend zumindest folgende Verfahrensschritte: a) Herstellung eines Rohlings des Bauteils mittels einem additiven Herstellungsverfahren oder Aufbringen mindestens eines ersten Bauteilelements auf ein zweites Bauteilelement mittels mindestens einem additiven Herstellungsverfahren zur Herstellung eines Rohlings des Bauteils; b) Einstellen einer vordefinierten Rauheit und/oder Güte von zumindest einem Teilbereich einer Oberfläche des Rohlings mittels Gleitschleifen; und c) Zerstörungsfreie Prüfung von zumindest dem Teilbereich der Oberfläche des Rohlings mit vordefinierter Rauheit und/oder Güte mittels einer Eindringprüfung, nämlich einer fluoreszierenden Eindringprüfung oder Farbeindringprüfung. Die Erfindung betrifft weiterhin die Verwendung des Verfahrens sowie Bauteile, die mit dem Verfahren hergestellt werden.

## Beschreibung

Die Erfindung betrifft ein Verfahren zur Fertigung eines Bauteils, insbesondere eines Bauteils einer Strömungsmaschine. Die Erfindung betrifft weiterhin die Verwendung des Verfahrens sowie Bauteile, die mit dem Verfahren hergestellt werden.

Verfahren zur Fertigung eines Bauteils, insbesondere eines Bauteils einer Strömungsmaschine sind in einer großen Vielzahl bekannt. Insbesondere sind additive oder generative Fertigungsverfahren (sog. Rapid Manufacturing- bzw. Rapid Prototyping-Verfahren) bekannt, bei denen das Bauteil durch pulverbettbasierte, additive Fertigungsverfahren schichtweise aufgebaut wird. Vorwiegend metallische Bauteile können beispielsweise durch Laser- bzw. Elektronenstrahlschmelz- oder -sinterverfahren hergestellt werden. Dabei wird zunächst schichtweise mindestens ein pulverförmiger Bauteilwerkstoff auf eine Bauteilplattform im Bereich einer Aufbau- und Fügezone der Vorrichtung aufgetragen. Anschließend wird der Bauteilwerkstoff schichtweise lokal verschmolzen und/oder versintert, indem dem Bauteilwerkstoff im Bereich der Aufbau- und Fügezone Energie mittels wenigstens eines Hochenergiestrahls, zum Beispiel eines Elektronen- oder Laserstrahls zugeführt wird. Der Hochenergiestrahl wird dabei in Abhängigkeit einer Schichtinformation der jeweils herzustellenden Bauteilschicht gesteuert. Nach dem Verschmelzen und/oder Versintern wird die Bauteilplattform schichtweise um eine vordefinierte Schichtdicke abgesenkt. Danach werden die genannten Schritte bis zur endgültigen Fertigstellung des Bauteils wiederholt. Aus dem Stand der Technik sind insbesondere auch generative Herstellverfahren für die Herstellung von Bauteilen einer Strömungsmaschine, wie beispielsweise von Bauteilen eines Flugtriebwerks oder einer Gasturbine bekannt, z.B. das in der DE 10 2009 051 479 A1 beschriebene Verfahren bzw. eine entsprechende Vorrichtung zur Herstellung eines Bauteils einer Strömungsmaschine. Bei diesem Verfahren wird durch schichtweisen Auftrag von mindestens einem pulverförmigen Bauteilwerkstoff auf einer Bauteilplattform im Bereich einer Aufbau- und Fügezone sowie schichtweises und lokales Schmelzen oder Sintern des Bauteilwerkstoffs mittels im Bereich der Aufbau- und Fügezone zugeführter Energie ein entsprechendes Bauteil hergestellt. Die Zufuhr der Energie erfolgt hierbei über Laserstrahlen, wie beispielsweise CO2-Laser, Nd:YAG-Laser, Yb-Faserlaser sowie Diodenlaser, oder durch Elektronenstrahlen.

Für die Überprüfung der Qualität zumindest der Oberflächenbereiche des additiv hergestellten Bauteils bzw. Bauteilrohlings ist es zunächst notwendig die Oberfläche des zunächst als Rohling hergestellten Bauteils auf eine geeignete Rauheit bzw. Oberflächengüte einzustellen. Nur dann ist es möglich, zerstörungsfreie Prüfverfahren für die Rissprüfung erfolgreich einzusetzen. Verfahren zur zerstörungsfreien Prüfung von Werkstückoberflächen sind ebenfalls bekannt. Bei der so genannten Eindringprüfung wird ein Eindringmittel auf eine gereinigte und zu überprüfende Werkstückoberfläche aufgebracht. Die Kapillarwirkung von feinen Oberflächenrissen und Poren begünstigt das Eindringen des Eindringmittels in derartige Ausnehmungen an der Werkstückoberfläche. Farbeindringprüfverfahren arbeiten mit nicht-fluoreszierenden Farblösungen als Eindringmittel (z.B. "Rot-Weiß-Verfahren"). Bei der so genannten fluoreszierenden Eindringprüfung wird mit einem fluoreszierenden Eindringmittel gearbeitet. Nach einer vorgegebenen Einwirkzeit wird dann überschüssiges Eindringmittel in einem Zwischenreinigungsvorgang abgewaschen. Anschließend wird ein Entwickler auf die zu prüfende Werkstückoberfläche aufgetragen. Der Entwickler fördert die Rückbenetzung des Eindringmittels an der Ausnehmung der Werkstückoberfläche, wobei durch den Entwickler das Eindringmittel aus der Ausnehmung an die Oberfläche gesaugt wird. Damit werden mögliche Unregelmäßigkeiten in der Werkstückoberfläche, wie z. B. rissartige Materialtrennungen deutlich sichtbar. Die fluoreszierende Eindringprüfung wird insbesondere im Flugzeug-, Schiffs- und Automobilbau sowie anderen metallverarbeitenden Industrien eingesetzt. Aber auch andere Stoffe, wie z. B. Keramik können auf entsprechende Oberflächenrisse und Poren untersucht werden. Um die benötigte Rauheit bzw. Oberflächengüte einzustellen werden additiv hergestellte Bauteile bzw. deren Rohlinge einem Nass-Strahlen unterzogen, da hierdurch insbesondere störende Hintergrundfluoreszenzen verringert werden können. Dieser Prozess ist jedoch sehr arbeits- und kostenintensiv.

Um die gewünschten Festigkeitseigenschaften von Nichtfunktionsflächen der Bauteile bzw. Bauteilrohlinge zu erhalten und um Zeichnungsforderungen an Oberflächenrauheiten zu erfüllen, werden diese zudem einer spanenden Nachbearbeitung mittels Werkzeugen definierter Scheidengeometrie (wie z.B. Fräsen, Drehen) unterzogen. Diese Nachbearbeitung erfolgt in den Prozessschritten nach dem Rissprüfen.

Nachteilig an dem diesem bekannten Verfahren ist jedoch, dass einerseits zur Vorbereitung des zerstörungsfreien Prüfverfahrens für die Rissprüfung ein arbeits- und kostenintensives Nass-Strahlverfahren verwendet wird und zudem eine spanende Nachbearbeitung zumindest der Nichtfunktionsflächen der Bauteile bzw. Bauteilrohlinge notwendig ist.

Aufgabe der vorliegenden Erfindung ist es, ein Verfahren zur Fertigung eines Bauteils, insbesondere eines Bauteils einer Strömungsmaschine zu schaffen, welches eine schnellere und kostengünstigere Fertigung des Bauteils ermöglicht. Des Weiteren ist es Aufgabe ein Bauteil einer Strömungsmaschine, insbesondere Bauteil eines Flugtriebwerks, bereitzustellen, welches schneller und kostengünstiger gefertigt werden kann.

Diese Aufgaben werden erfindungsgemäß durch ein Verfahren mit den Merkmalen des Patentanspruchs 1, einer Verwendung des Verfahrens gemäß den Merkmalen des Anspruchs 11 und ein Bauteil mit den Merkmalen des Anspruchs 12 gelöst. Vorteilhafte Ausgestaltungen mit zweckmäßigen Weiterbildungen der Erfindung sind in den jeweiligen Unteransprüchen angegeben.

Ein erster Aspekt der Erfindung betrifft ein Verfahren zur Fertigung eines Bauteils, insbesondere eines Bauteils einer Strömungsmaschine, umfassend zumindest folgende Verfahrensschritte:
a) Herstellung eines Rohlings des Bauteils mittels einem additiven Herstellungsverfahren oder Aufbringen mindestens eines ersten Bauteilelements auf ein zweites Bauteilelement mittels mindestens einem additiven Herstellungsverfahren zur Herstellung eines Rohlings des Bauteils;
b) Einstellen einer vordefinierten Rauheit und/oder Güte von zumindest einem Teilbereich einer Oberfläche des Rohlings mittels Gleitschleifen; und
c) Zerstörungsfreie Prüfung von zumindest dem Teilbereich der Oberfläche des Rohlings mit vordefinierter Rauheit und/oder Güte mittels einer Eindringprüfung, nämlich einer fluoreszierenden Eindringprüfung oder Farbeindringprüfung.

Das erfindungsgemäße Verfahren ermöglicht es, dass die Fertigung des Bauteils schneller und kostengünstiger durchgeführt werden kann. So kann zur Vorbereitung für die Rissprüfung auf das arbeits- und kostenintensive Nass-Strahlverfahren verzichtet werden. Die für die Rissprüfung notwendige Oberflächenrauheit und/oder Oberflächengüte des Rohlings bzw. des Bauteils wird mittels dem kostengünstigeren Gleitschleifverfahren erzielt. Zudem ergibt sich durch den Zwischenschritt des Gleitschleifens der Vorteil, dass zumindest auf eine spanende Nachbearbeitung der Nichtfunktionsflächen des zu fertigenden Bauteils verzichtet werden kann, da die gewünschten Festigkeitseigenschaften von Nichtfunktionsflächen bereits durch das Gleitschleifen erzielt werden können.

In einer weiteren vorteilhaften Ausgestaltung des erfindungsgemäßen Verfahrens wird vor dem Einstellen der vordefinierten Rauheit und/oder Güte von zumindest dem Teilbereich der Oberfläche des Rohlings gemäß Verfahrensschritt b) eine Wärmebehandlung des mittels des additiven Verfahrens hergestellten Rohlings des Bauteils durchgeführt. Dadurch können Eigenspannungen im hergestellten Rohling reduziert bzw. beseitigt werden, was wiederum eine erhöhte Qualität des zu fertigenden Bauteils gewährleistet.

In weiteren vorteilhaften Ausgestaltungen des erfindungsgemäßen Verfahrens ist das additive Herstellungsverfahren ein selektives Laserstrahlschmelzverfahren, ein Elektronenstrahlschmelzverfahren, ein selektives Laserstrahlsinterverfahren, ein Elektronenstrahlsintemerfahren oder ein Kaltgasspritzverfahren. Weitere additive bzw. generative Herstellungsverfahren sind denkbar. Die Verwendung von additiven Herstellungsverfahren ermöglicht eine schnelle und kostengünstige Fertigung von Bauteilen, insbesondere von Bauteilen mit konstruktiv anspruchsvollen Geometrien. Dabei kann zur Herstellung des Rohlings des Bauteils gemäß Verfahrensschritt a) mindestens ein pulverförmiger oder pastöser, metallischer oder keramischer Bauteilwerkstoff verwendet werden.

In einer weiteren vorteilhaften Ausgestaltung des erfindungsgemäßen Verfahrens wird im Verfahrensschritt b) der Rohling des Bauteils innerhalb eines mit Schleifkörpern gefüllten Trogs bzw. Arbeitsraums einer Gleitschleifvorrichtung, mittels welcher jeweilige Oberflächen des Rohlings unter Relativbewegung zwischen dem Bauteil und den Schleifkörpern abschleifbar sind, angeordnet und bewegt. Dabei genügt es, dass die Bauteile bzw. Bauteilrohlinge lose in den Trog bzw. Arbeitsraum der Gleitschleifvorrichtung eingebracht werden und sich mit dem Fluss der Schleifkörper bewegen. Es ist allerdings auch denkbar, dass das Bauteil zunächst in einer Einspannvorrichtung befestigt wird, wobei die Einspannvorrichtung mit dem Bauteil dann in den Trog bzw. Arbeitsraum der Gleitschleifvorrichtung eingebracht wird. Die verwendeten Schleifkörper können dabei aus Keramik, Stahl oder Korund bestehen. Auch andere Materialien für die Ausgestaltung geeigneter Schleifkörper sind denkbar.

In einer vorteilhaften Ausgestaltung des erfindungsgemäßen Verfahrens erfolgt nach der zerstörungsfreien Prüfung des Rohlings gemäß Verfahrensschritt c) eine optische Auswertung der Oberfläche des Rohlings. Dadurch können mögliche untypische Ausnehmungen bzw. Fehlstellen in der Bauteiloberfläche aufgefunden werden. Die optische Auswertung kann bei Verwendung eines fluoreszierenden Eindringmittels mittels Bestrahlung der zu überprüfenden Bauteiloberfläche mit UV-Licht erfolgen. Dadurch können mit dem fluoreszierenden Eindringmittel ausgefüllte bzw. benetzte Ausnehmungen an der Bauteiloberfläche deutlich erkannt werden. Durch die verwendete UV-Strahlung ist eine deutliche und exakte Sichtbarmachung von Ausnehmungen in der Bauteiloberfläche möglich.

In einer weiteren vorteilhaften Ausgestaltung des erfindungsgemäßen Verfahrens weist zumindest der mittels Gleitschleifen bearbeitete Teilbereich der Oberfläche des Rohlings nach dem Gleitschleifen eine gemittelte Rautiefe von E_{z} ≤ 9,0 µm auf. Die Oberflächengüte bzw. Oberflächenrauheit erfüllt damit vorteilhafterweise die geforderten Festigkeitseigenschaften von Nichtfunktionsflächen bestimmter Bauteile im Triebwerksbau und zum anderen die Anforderungen zur Durchführung einer erfolgreichen Rissprüfung mittels der Eindringprüfung.

In einer weiteren vorteilhaften Ausgestaltung des erfindungsgemäßen Verfahrens ist das Bauteil ein Boroskopauge oder Endoskopauge. Durch das erfindungsgemäße Verfahren können diese Bauteile kostengünstig in der erforderlichen Qualität hergestellt werden. Insbesondere kann es sich dabei um Boroskopaugen handeln, die als Teil eines Turbinengehäuses eines Flugtriebwerks eingesetzt werden.

Gemäß einem zweiten Aspekt der Erfindung wird das im Vorhergehenden beschriebene erfindungsgemäße Verfahren insbesondere bei der Herstellung und bei der Instandsetzung von Bauteilen einer Strömungsmaschine, insbesondere von Bauteilen einer Gasturbine, verwendet. Die sich aus der Verwendung des Verfahrens gemäß dem ersten Erfindungsaspekt ergebenden Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

Ein dritter Aspekt der Erfindung betrifft ein Bauteil einer Strömungsmaschine, insbesondere ein Bauteil eines Flugtriebwerks hergestellt nach einem Verfahren wie im Vorhergehenden beschrieben. Vorteilhafterweise kann dieses Bauteil im Vergleich zu den im Stand der Technik bekannten Verfahren schneller und kostengünstiger unter Erfüllung der erforderlichen Qualitätskriterien hergestellt werden.

In einer vorteilhaften Ausgestaltung des erfindungsgemäßen Bauteils weist das Bauteil zumindest in Teilbereichen eine Oberfläche mit einer gemittelten Rautiefe von R_{z} ≤ 9,0 µm auf. Damit können trotz der schnelleren und kostengünstigeren Fertigung des Bauteils die Anforderungen an die Oberflächengüte bzw. Oberflächenrauheit im Hinblick auf die Festigkeitseigenschaften der Nichtfunktionsflächen des fertigen Bauteils, insbesondere von Bauteilen im Triebwerksbau, erfüllt werden, indem zumindest die Nichtfunktionsflächen des Bauteils eine gemittelte Rautiefe von R_{z} ≤ 9,0 µm aufweisen. Bei dem erfindungsgemäßen Bauteil kann es sich um ein Boroskopauge oder Endoskopauge, insbesondere zur Verwendung in Flugtriebwerken handeln.

Die sich aus der Verwendung des Verfahrens gemäß dem ersten Erfindungsaspekt ergebenden Merkmale und Vorteile sind den Beschreibungen des ersten Erfindungsaspekts zu entnehmen, wobei vorteilhafte Ausgestaltungen des ersten Erfindungsaspekts als vorteilhafte Ausgestaltungen des dritten sowie des zweiten Erfindungsaspekts und umgekehrt anzusehen sind.

Weitere Merkmale der Erfindung ergeben sich aus den Ansprüchen, dem Ausführungsbeispiel sowie anhand der Zeichnung. Die vorstehend in der Beschreibung genannten Merkmale und Merkmalskombinationen sowie die nachfolgend in dem Ausführungsbeispiel genannten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Dabei zeigt

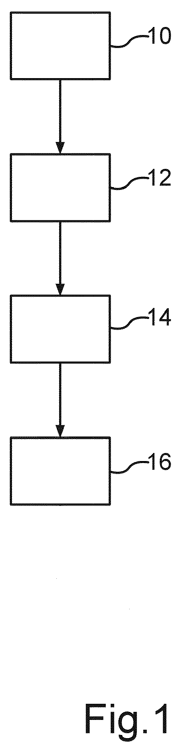

Fig. 1 ein Ablaufschema eines erfindungsgemäßen Verfahrens zur Fertigung von Bauteilen.

Fig. 1 zeigt ein Ablaufschema eines Verfahrens zur Fertigung eines Bauteils, insbesondere eines Bauteils einer Strömungsmaschine. In dem dargestellten Ausführungsbeispiel ist das Bauteil ein Boroskopauge zur Verwendung in einem Flugtriebwerk. Dabei wird in einem ersten Verfahrensschritt 10 ein Rohling des Bauteils mittels einem additiven Herstellungsverfahren hergestellt. Das additive Herstellungsverfahren ist dabei ein selektives Laserstrahlschmelzen, wobei als Werkstoff für das Bauteil bzw. den Rohling des Bauteils Metallpulver bereitgestellt wird, welches mittels Laserenergie schichtweise zur Ausgestaltung des Bauteils bzw. Rohlings miteinander verschmolzen wird.

In einem folgenden Verfahrensschritt 12 wird der Rohling des Bauteils einer Wärmebehandlung unterzogen. Die Wärmebehandlung dient zur Reduzierung bzw. Beseitigung von Eigenspannungen im Bauteil bzw. Bauteilrohling.

Nach der Wärmebehandlung des Bauteilrohlings wird in einem folgenden Verfahrensschritt 14 die Rauheit und/oder Güte der Oberfläche des Rohlings mittels Gleitschleifen eingestellt. Die vordefinierte Rauheit weist in dem dargestellten Ausführungsbeispiel eine gemittelte Rautiefe von R_{z} ≤ 9,0 µm auf. Um diese Rauheit zu erreichen, wird das Bauteil bzw. der Rohling des Bauteils in einen mit Schleifkörpern gefüllten Trog einer Gleitschleifvorrichtung verbracht. Unter Relativbewegung zwischen dem Bauteil und den Schleifkörpern werden die jeweiligen Oberflächen des Rohlings bis zur gewünschten Rauheit bzw. Güte abgeschliffen.

In einem abschließenden Verfahrensschritt 16 erfolgt eine zerstörungsfreie Prüfung der Oberfläche des Rohlings mittels einer Eindringprüfung, nämlich einer fluoreszierenden Eindringprüfung oder Farbeindringprüfung. Aufgrund der Eindringprüfung kann festgestellt werden, ob das Bauteil den Qualitätsanforderungen genügt. Sollte das Bauteil den Qualitätsanforderungen genügen, so besteht zudem die Möglichkeit, dass Funktionsflächen des Bauteils einer spanenden Nachbearbeitung zugeführt werden. Die Nichtfunktionsflächen des Bauteils erfüllen bereits aufgrund der eingestellten, vordefinierten Rauheit den Qualitätsanforderungen, insbesondere den Festigkeitsanforderungen an diese Bereiche, so dass hier keine spanende Nachbearbeitung mehr notwendig ist.

### Bezugszeichenliste

- 10: Verfahrensschritt
- 12: Verfahrensschritt
- 14: Verfahrensschritt
- 16: Verfahrensschritt

## Patentansprüche

1. Verfahren zur Fertigung eines Bauteils, insbesondere eines Bauteils einer Strömungsmaschine, umfassend zumindest folgende Verfahrensschritte:
a) Herstellung eines Rohlings des Bauteils mittels einem additiven Herstellungsverfahren oder Aufbringen mindestens eines ersten Bauteilelements auf ein zweites Bauteilelement mittels mindestens einem additiven Herstellungsverfahren zur Herstellung eines Rohlings des Bauteils;
b) Einstellen einer vordefinierten Rauheit und/oder Güte von zumindest einem Teilbereich einer Oberfläche des Rohlings mittels Gleitschleifen; und
c) Zerstörungsfreie Prüfung von zumindest dem Teilbereich der Oberfläche des Rohlings mit vordefinierter Rauheit und/oder Güte mittels einer Eindringprüfung, nämlich einer fluoreszierenden Eindringprüfung oder Farbeindringprüfung.

2. Verfahren nach Anspruch 1, **dadurch gekennzeichnet, dass** vor dem Einstellen der vordefinierten Rauheit und/oder Güte von zumindest dem Teilbereich der Oberfläche des Rohlings gemäß Verfahrensschritt b) eine Wärmebehandlung des mittels des additiven Verfahren hergestellten Rohlings des Bauteils erfolgt.

3. Verfahren nach Anspruch 1 oder 2, **dadurch gekennzeichnet, dass** das additive Herstellungsverfahren ein selektives Laserstrahlschmelzverfahren, ein Elektronenstrahlschmelzverfahren, ein selektives Laserstrahlsinterverfahren, ein Elektronenstrahlsinterverfahren oder ein Kaltgasspritzverfahren ist.

4. Verfahren nach einem der vorhergehenden Ansprüche, **dadurch gekennzeichnet, dass** zur Herstellung des Rohlings des Bauteils gemäß Verfahrensschritt a) mindestens ein pulverförmiger oder pastöser, metallischer oder keramischer Bauteilwerkstoff verwendet wird.

5. Verfahren nach einem der vorhergehenden Ansprüche, **dadurch gekennzeichnet, dass** im Verfahrensschritt b) der Rohling des Bauteils innerhalb eines mit Schleifkörpern gefüllten Troges bzw. Arbeitsraums einer Gleitschleifvorrichtung, mittels welcher jeweilige Oberflächen des Rohlings unter Relativbewegung zwischen dem Bauteil und den Schleifkörpern abschleifbar sind, angeordnet und bewegt wird.

6. Verfahren nach Anspruch 5, **dadurch gekennzeichnet, dass** die Schleifkörper aus Keramik, Stahl oder Korund bestehen.

7. Verfahren nach einem der vorhergehenden Ansprüche, **dadurch gekennzeichnet, dass** nach der zerstörungsfreien Prüfung des Rohlings gemäß Verfahrensschritt c) eine optische Auswertung der Oberfläche des Rohlings erfolgt.

8. Verfahren nach Anspruch 7, **dadurch gekennzeichnet, dass** die optische Auswertung mittels Bestrahlung der zu überprüfenden Oberfläche mit UV-Licht erfolgt.

9. Verfahren nach einem der vorhergehenden Ansprüche, **dadurch gekennzeichnet, dass** zumindest der mittels Gleitschleifen bearbeitete Teilbereich der Oberfläche des Rohlings nach dem Gleitschleifen eine gemittelte Rautiefe von R_{z} ≤ 9,0 µm aufweist.

10. Verfahren nach einem der vorhergehenden Ansprüche, **dadurch gekennzeichnet, dass** das Bauteil ein Boroskopauge oder Endoskopauge ist.

11. Verwendung eines Verfahrens nach einem der Ansprüche 1 bis 10 bei der Herstellung oder Instandsetzung von Bauteilen einer Strömungsmaschine, insbesondere von Bauteilen eines Flugtriebwerks.

12. Bauteil einer Strömungsmaschine, insbesondere Bauteil eines Flugtriebwerks hergestellt nach einem Verfahren gemäß den Ansprüchen 1 bis 10.

13. Bauteil einer Strömungsmaschine nach Anspruch 12, **dadurch gekennzeichnet, dass** das Bauteil zumindest in Teilbereichen eine Oberfläche mit einer gemittelten Rautiefe von R_{z} ≤ 9,0 µm aufweist.

14. Bauteil einer Strömungsmaschine nach Anspruch 12 oder 13, **dadurch gekennzeichnet, dass** zumindest Nichtfunktionsflächen des Bauteils eine gemittelten Rautiefe von R_{z} ≤ 9,0 µm aufweisen.

15. Bauteil einer Strömungsmaschine nach einem der Ansprüche 12 bis 14, **dadurch gekennzeichnet, dass** das Bauteil ein Boroskopauge oder Endoskopauge ist.
